# EUROPEAN PATENT SPECIFICATION

(11) **EP 1 270 007 B1**
(45) Date of publication and mention of the grant of the patent: **26.05.2004**
(21) Application number: 02013398.9
(22) Date of filing: 19.06.2002
(51) Int. Cl.: A61K 31/485, A61K 9/107, A61P 25/16

(54) **Microemulsions for the transdermal administration of apomorphine, for the treatment of Parkinson's disease**
Mikroemulsionen zur transdermalen Verabreichung von Apomorphin für die Behandlung von Morbus Parkinson
Microemulsions pour l'administration transdermale d'apomorphine pour le traitement de la maladie de Parkinson

(30) Priority: 22.06.2001 IT MI20011321
(43) Date of publication of application: 02.01.2003
(73) Proprietor: CHIESI FARMACEUTICI S.p.A., I-43100 Parma (IT)
(72) Inventor: Gasco, Maria Rosa, 43100 Parma (IT)
(74) Representative: Minoja, Fabrizio, Dr.

(56) References cited:
- FR-A- 2 732 896
- US-A- 5 562 917
- DATABASE CA [Online] CHEMICAL ABSTRACTS SERVICE, COLUMBUS, OHIO, US; LEMACHATTI, S. ET AL: "Feasibility of percutaneous apomorphine delivery by a transdermal therapeutic system for Parkinson's disease treatment" retrieved from STN Database accession no. 127:166691 HCA XP002222677 & ACTA TECHNOLOGIAE ET LEGIS MEDICAMENTI (1996), 7(3), 231-238 ,
- DATABASE PASCAL [Online] INIST, CNRS (CENTRE NATIONAL DE LA RECHERCHE SCIENTIFIQUE), VANDOEUVRE-LES-NANCY, FR; CONTIN MANUELA ET AL: retrieved from STN Database accession no. 2001-0047901 XP002222678 & CNS DRUGS, (2000), 14(6), 439-455, 147 REFS. ISSN: 1172-7047, Laboratory of Clinical Neuropharmacology, Institute of Neurology, University of Bologna, Bologna, Italy
- DATABASE SCISEARCH [Online] SCHELOSKY L ET AL: "CURRENT STRATEGIES IN THE DRUG-TREATMENT OF ADVANCED PARKINSONS-DISEASE - NEW MODES OF DOPAMINE SUBSTITUTION" retrieved from STN Database accession no. 412373 XP002222679 & ACTA NEUROLOGICA SCANDINAVICA, (1993) VOL. 87, SUPP. 146, PP. 46-49. ISSN: 0001-6314., FREE UNIV BERLIN, UKRV, DEPT NEUROL, AUGUSTENBURGER PL 1, W-1000 BERLIN 65, GERMANY

## Description

The present invention relates to pharmaceutical compositions comprising apomorphine or salts thereof in the form of microemulsions.

### PRIOR ART

Apomorphine, ((R)-5,6,6a,7-tetrahydro-6-methyl-4H-dibenzo[de,g]-quinoline-10,11-diol) is a dopaminergic drug, used for the treatment of patients with Parkinson's disease due to its therapeutic activity higher than other active principles. Since it can also be administered in comparatively high doses, apomorphine reduces daily mean immobility/rigidity in several patients even in the most advanced steps of the disease.

At present, the most used administration route is by far the subcutaneous, which in the most severe cases is carried out by continuous infusion with penjects or minipumps.

Some of the drawbacks of said administration, especially in the case of infusions, are that the patient is almost immobilized during the whole administration period and that nodules frequently form at the needle insertion site.

Analogously, the oral administration route is indeed unsatisfactory, due to the high emetic effect of apomorphine.

Studies on alternative administration routes, such as the rectal, sublingual, nasal routes, have been carried out. Moreover, a composition for topical use in the form of a cream containing apomorphine is reported in literature; nevertheless, after use of said composition no detectable amounts of said substance could be found in the plasma (S.T.Gancher, J.G.Nutt, W.R.Woodward, Movement disorders -vol 6, 212,1991).

In order to improve mobility during the whole day as well as life quality in Parkinson patients, the active ingredient has to be administered continuously and constantly in such a way as to ensure a steady stimulation of the dopaminergic receptors. At this regard, the transdermal route could be particularly suitable.

Therefore it would be highly advantageous to provide a novel formulation for apomorphine which can be delivered by said route of administration and able to provide a continuous and constant release over time, without undesired effects. It would be even more advantageous to provide a formulation compatible with the physiological pH values of the skin in whose microenvironment apomorphine is stable.

### SUMMARY OF THE INVENTION

It has now been found that a pharmaceutical composition suitable for the transdermal administration can be obtained by solubilizing apomorphine, or a salt thereof, in a particular microemulsion, which overcomes the drawbacks of the known technique.

Therefore the object of the present invention is a pharmaceutical composition comprising apomorphine, or the salts thereof, suitable for the transdermal administration, characterized in that the carrier for apomorphine is in the form of microemulsion.

The characteristics and the advantages of the apomorphine pharmaceutical composition according to the present invention and of the related preparation process will be shown in detail in the following disclosure.

### DETAILED DISCLOSURE OF THE INVENTION

Microemulsions are thermodynamically stable dispersions of water and oil, usually stabilized by one or more surfactants and by one or more cosurfactants.

The process for the preparation of the microemulsions of the present invention comprises the following steps:
a) preparing an aqueous mixture containing one or more cosurfactants and one or more hydrophilic antioxidants, and optionally, one or more surfactants;
b) preparing a mixture containing at least one oil, at least one surfactant, one or more cosurfactants, optionally other adjuvants such as propylene glycol andone or more lipophilic antioxidants
c) adding apomorphine, in the form of the hydrochloride or other salt, to the aqueous mixture, or in the form of the free base to the oily mixture;
d) contacting the oily mixture with the aqueous mixture under gentle stirring and at room temperature, to obtain a clear system;
e) optionally adding a viscosizing agent and an absorption promoter to the microemulsion obtained in step d).

The pH of the aqueous mixture and of the microemulsion typically ranges between 5.4 and 6.2 and is preferably in the range 5.6 - 6.0.

When apomorphine hydrochloride is added to the aqueous mixture a), the pH of the microemulsion is adjusted, if necessary, to said range of values by addition of a diluted NaOH aqueous solution, in appropriate amount.

When apomorphine as free base is added to the oily mixture b), the pH of the microemulsion is adjusted, if necessary, to said values by addition of a diluted HCl solution.

It has been surprisingly found that, contrary to the teaching of the prior art, apomorphine in the microenvironment of the formulations of the invention remains stable at pH values far away from those (3-4) which are considered optimal for preventing and/or retarding the oxidation of the cathecol group so the formation of degradation products (The Pharm. Codex 12^{th} Ed. 1994, pp 739-741; Lundgren et al Acta Pharm Suecica 1970, 7 133-148; GB 2343376). On the other hand, the adjustment of the pH to more physiological values makes the formulation well tolerated and prevents possible skin irritation effects.

It has also been found that said pH range is optimal for stabilizing the ion-pair between the drug and the medium aliphatic chain carboxylic acid, one of the utilized cosurfactant, and thus to increase the lipophilicity of the whole molecule. High lipophilicity, in turn is an important requirement for transdermal therapy as it gives rise to an increment of the amount and the rate of the drug delivered.

Apomorphine base or physiologically acceptable salts thereof can be used as active ingredient for the formulations of the invention, advantageously in concentration between 0.5 and 10%, preferably between 1.0 and 5.0% based on the total weight of the formulation.

Advantageously, the microemulsions of the invention could be in the form of w/o or o/w emulsions, preferably w/o wherein the active ingredient as hydrochloride salt is dissolved in the aqueous phase.

They contain nanodroplets of the dispersed phase having a diameter ranging from 60 to 100 nm, preferably from 70 to 90 nm, and a polydispersity index ranging from 0.10 to 0.35. In the microemulsions of the invention the apomorphine can be present in combination with other active ingredients also useful for the treatment of Parkinson's disease and in particular with other agents useful for reducing the skin irritation effects, for example antiinflammatory steroids such as dexamethasone.

The characterization of the microemulsions diameters was carried out by photocorrelation spectroscopy with N 4 Coulter instrumentation.

The oil phase used in the preparation of the microemulsions of the invention is selected from aliphatic alcohols, esters of aliphatic alcohols, glycerol esters and mixtures thereof.

According to a preferred embodiment of the present invention, the oil is selected from medium chain aliphatic alcohols and esters thereof, in particular decanol, dodecanol, isopropyl myristate and mixtures thereof.

The surfactants used for the preparation of the microemulsions of the invention are selected from polyoxyethylene sorbitans, such as Tween 20, Tween 40, Tween 60, polyoxyethylene castor oil derivatives, such as Cremophor EL, soy lecithin, purified egg lecithin, phospholipids and mixtures thereof.

The cosurfactants and the other adjuvants used for the preparation of the microemulsions of the invention are selected from alcohols and glycols, such as butanol, hexanol, benzyl alcohol, cyclohexanol, phenyl ethyl alcohol, propylene glycol, hexanediol, bile salts such as taurocholic and glycocolic, medium aliphatic chain carboxylic acids (C₄-C₁₂) such as butyric, hexanoic, octanoic acid or salts thereof, monoalkyl phosphates such as octyl phosphate and hexadecyl phosphate. In a preferred embodiment of the invention, the cosurfactant is selected from hexanoic acid, octanoic acid, sodium taurocholate, sodium glycocholate or mixtures thereof. The antioxidant agents used for preserving the present microemulsions can be of lipophilic nature, for example δ-tocopherol, ascorbyl palmitate, and/or of hydrophilic nature, for example ascorbic acid.

According to a preferred embodiment of the present invention, the lipophilic antioxidant agents are added to the oily mixture in concentrations ranging from 0.2 to 2.0 % by weight, whereas the hydrophilic antioxidants are added to the aqueous mixture in concentration ranging from 0.2 to 0.8% by weight.

The optional viscosity-increasing agent, added to increase the microemulsion viscosity, is selected from a Carbomer Carbopol 941 and silicon dioxide, for example Aerosil 200.

Any optional absorption promoters are selected from glyceryl monooleate, limonene, and decyl methyl sulphoxide.

Preferred microemulsions according to the present invention have the following composition:

| | % by weight |
|---|---|
| Apomorphine | 1.0-5.0 |
| Decanol | 8.0-15.0 |
| Dodecanol | 0.0-4.0 |
| Propylene glycol | 2.0-20 |
| Isopropyl myristate | 0-30 |
| Butanol | 0-8.0 |
| Benzyl alcohol | 0-12.0 |
| Phenyl ethyl alcohol | 4.0-9.0 |
| Taurocholate | 0-7.0 |
| Glycocholate | 0-7.0 |
| Cremophor EL | 20-26 |
| Tween 20 | 17-25 |
| Butyric acid | 1-4 |
| Hexanoic acid | 0-7 |
| Octanoic acid | 0-8 |
| Monooctyl phosphate | 0-5.0 |
| Aerosil 200 | 0-7.0 |
| Soy lecithin | 6-12 |
| Water | q.s. to obtain 100 parts by weight of microemulsion |

The microemulsions of the invention allow to dissolve an amount of apomorphine sufficient to obtain the desired therapeutic effect, and to vehicle it through the skin corneous layer with an induction time of about 2-4 hours, thereby providing transdermal administration.

Through this administration route, which is alternative to those of the known technique, the compositions of the invention also provide the continuous release of comparatively high amounts of apomorphine (10-100 mg daily, preferably 30-60 mg daily), without need for subcutaneous infusions. Moreover, the compositions of the invention allow patients capacity of movement for the whole administration period.

The formulations of the inventions are advantageously incorporated in matrix- or reservoir-type patches for transdermal administration. Preferably the patch is a reservoir type and typically comprises an impermeable backing layer which is sealed at its periphery to a rate-controlling membrane layer thus defining a drug depot in which the formulation is contained. More preferably, the drug depot is in the form of multiple reservoir in order to allow the patient to adjust the dose according to its need.

The pharmaceutical compositions of the invention are useful in all the treatments wherein the administration of apomorphine is required, in particular in the treatment of Parkinson's disease.

The present invention is illustrated in more detail in the following preparation examples.

### Example 1

A microemulsion according to the invention is prepared by the following procedure:
a) water (38 g) added with 0.4% ascorbic acid, propylene glycol (17 g) and Cremophor EL (25 g) are mixed;
b) a mixture consisting of 9 g of decanol, 1.0 g of dodecanol added with 1% ascorbyl palmitate, butyric acid (2.0 g), phenylethyl alcohol (6.0 g) is prepared;
c) 2 g of apomorphine as free base are added to the mixture from step b);
d) the oily mixture from step b) is added to the mixture from step a) under gentle stirring, to obtain a clear system.

All the steps are carried out at room temperature.

The pH value of the microemulsion is measured and adjusted to 5.8 by addition of a diluted NaOH solution.

Photocorrelation spectroscopy gave the following results: nanodroplets mean diameter: 80 nm, polydispersity index: 0.23

### Example 2

A microemulsion according to the invention is prepared by the following procedure:
a) a mixture consisting of water (36 g) added with 0.2% of ascorbic acid, propylene glycol (16 g) and Tween 20 (24 g) is prepared;
b) a mixture consisting of 8.5 g of decanol added with 1% of ascorbyl palmitate, 6.0 g of benzyl alcohol and 2.0 g of octanoic acid is prepared;
c) 2.5 g of apomorphine free base are added to the mixture from step b);
d) the oily mixture from step b) is added to the mixture from step a), under gentle stirring to obtain a clear system;
e) 6.0 g of the viscosity-increasing agent Aerosil 200 are added under stirring to the resulting microemulsion.

The pH value of the microemulsion is measured and adjusted to 5.8 by addition of a diluted NaOH solution.

All the steps are carried out at room temperature.

Photocorrelation spectroscopy (carried out before viscosization) gave the following results: mean nanodroplets diameter: 90 nm, polydispersity index: 0.20.

### Example 3

A microemulsion according to the invention is prepared by the following procedure:
a) a mixture consisting of water (20 g) added with 0.8% of ascorbic acid, sodium taurocholate (6.0 g), butyric acid (1.5) is prepared;
b) a mixture consisting of 20 g of decanol and 32 g of isopropyl myristate added with 1% of ascorbyl palmitate, 9.5 g of soy lecithin (Epikuron 200) and 6 g of benzyl alcohol is prepared;
c) 1.0 g of apomorphine hydrochloride are added to the mixture from step a); the pH of the solution is adjusted to 5.6 by adding small aliquots of a NaOH solution;
d) the aqueous mixture from step a) is added to the oily mixture from step b), under gentle stirring, to obtain a clear system.

All the steps are carried out at room temperature.

Photocorrelation spectroscopy gave the following results: mean diameter of the nanodroplets: 70 nm, polydispersity index: 0.23.

### Example 4

A microemulsion according to the invention is prepared by the following procedure:
a) a mixture consisting of water (18.4 g) added with 0.5% of ascorbic acid, apomorphine hydrochloride (3.9 g), 1,2 propanediol (4.4 g), octanoic acid (4.7), sodium hexanoate (3.6 g), sodium glycocholate (4.7 g) is prepared and pH is adjusted to 6.0;
b) an oily mixture consisting of Epikuron 200 (7.4 g), isopropyl myristate (25.5 g), decanol (17.0 g) benzyl alcohol (10.4 g) is prepared;
c) the oily mixture from step b) is added under gentle stirring to the aqueous mixture from step a) until a clear system is obtained;
d) 5.9 g of Aerosil 200 are then added and mixing is continued until homogeneous viscosity.

### Example 5

A microemulsion according to the invention is prepared by the following procedure:
a) a mixture of water added with 0.2% of ascorbic acid (18.2 g), apomorphine hydrochloride (3.9 g), octanoic acid (4.6 g), sodium octanoate (3.5 g), 1,2-propanediol (7.6 g), sodium taurocholate (5.7 g) is prepared and adjusted to pH 6.0;
b) an oily mixture consisting of Epikuron 200 (7.3 g), isopropyl myristate (25.3 g), decanol (16.8 g), benzyl alcohol (7.1 g) is prepared;
c) the oily mixture (from step b) is added to the aqueous mixture from step a) under gentle stirring until a clear system is obtained and then 5.9 g of Aerosil 200 are added.

### Example 6

### Experimentation on apomorphine permeation through nude mouse skin

Permeation studies were carried out on the skin of hairless mice aged 4-5 weeks and weighing 25-30 g using vertical Franz cells. The experimentation was performed on hairless mouse skin, (rinsed with normal saline), which was placed in the cell so as to delimit two parts, a receptor compartment containing a solution as defined hereinafter, for receiving the medicament permeating through said skin, and a donor part (on top of which the skin and microemulsion are placed). The receptor cell - of 6 mL capacity - was filled up with a buffer solution at pH 4.0, added with ascorbic acid in amount of 0.4%; said solution is always kept under stirring. The cell is thermostatized at 37°C.

A microemulsion according to Example 1 reported above was prepared. 400 mg of this microemulsion are applied to 2 cm² of hairless mouse skin prepared as reported above, on the side facing the empty part of the cell.

At 1-hour intervals, the entire content of the receptor cell is removed and replaced with fresh buffer. The amount of apomorphine permeated through hairless mouse skin in the receptor solution was determined by means of HPLC analysis with UV Detector (λ=273 nm) on the solution at appropriate intervals. Said analysis, carried out for 24 hours, allowed to observe an induction time of some hours, thereafter the flux was kept constant for the remaining 19 hours.

Based on HPLC analytical data, the flux at steady-state was calculated, i.e. the amount of medicament, which permeates per hour through 1 cm² of skin, once exceeded the induction time, and a value of 0.100 mg/h/cm² was obtained.

## Claims

1. A microemulsion containing apomorphine or salts thereof, suitable for transdermal administration.

2. A microemulsion as claimed in claim 1, consisting of:
a) an aqueous mixture containing one or more cosurfactants and one or more hydrophilic antioxidants and, optionally, one or more surfactants;
b) a mixture containing at least one oil, at least one surfactant, one or more cosurfactants,one or more lipophilic antioxidants and other adjuvants;
c) apomorphine, in the form of hydrochloride or other salt;
d) optionally, viscosity-increasing agents and absorption enhancers.

3. A microemulsion as claimed in the above claims, wherein pH ranges from 5.4 to 6.2.

4. A microemulsion as claimed in the above claims, wherein pH is in the range 5.6 - 6.0.

5. A microemulsion as claimed in the above claims, wherein the nanodrops of the dispersed phase have diameter ranging from 60 to 100 nm.

6. A microemulsion as claimed in the above claims, wherein the nanodrops of the dispersed phase have diameter ranging from 70 to 90 nm.

7. A microemulsion as claimed in the above claims, wherein the nanodrops have polydispersity index ranging from 0.10 to 0.35.

8. A microemulsion as claimed in the above claims, wherein the oil is selected from aliphatic alcohols, esters of aliphatic alcohols, glycerol esters and mixtures thereof.

9. A microemulsion as claimed in claim 8, wherein the oil is selected from medium chain aliphatic alcohols and esters thereof, in particular decanol, dodecanol, isopropyl myristate and mixtures thereof.

10. A microemulsion as claimed in the above claims, wherein the surfactants are selected from polyoxyethylene sorbitans, polyoxyethylene castor oil derivatives, soy lecithin, purified egg lecithin, phospholipids and mixtures thereof.

11. A microemulsion as claimed in claim 10, wherein the surfactants are selected from Tween 20, Tween 40, Tween 60, Cremophor EL.

12. A microemulsion as claimed in the above claims, wherein the cosurfactants are selected from alcohols and glycols, such as butanol, hexanol, benzyl alcohol, cyclohexanol, phenylethyl alcohol, propylene glycol, hexanediol, bile salts such as taurocholic and glycocolic, carboxylic acids such as butyric, hexanoic, octanoic acids or salts thereof, monoalkyl phosphates such as octyl phosphate and hexadecyl phosphate.

13. A microemulsion as claimed in claim 12, wherein cosurfactants are selected from butanol, hexanol, benzyl alcohol, cyclohexanol, phenylethyl alcohol, propylene glycol, hexanediol.

14. A microemulsion as claimed in claim 12, wherein cosurfactants are selected from taurocholic and glycocolic.

15. A microemulsion as claimed in claim 12, wherein cosurfactants are selected from butyric, hexanoic, octanoic acid or salts thereof.

16. A microemulsion as claimed in the above claims, wherein the antioxidants agents are of lipophilic and/or hydrophilic nature.

17. A microemulsion as claimed in claim 16, wherein the antioxidants agents are δ-tocopherol, ascorbyl palmitate, ascorbic acid.

18. A microemulsion as claimed in the above claims, wherein lipophilic antioxidant agents are added in concentrations ranging from 0.2 to 2.0 % by weight, and hydrophilic antioxidants are added in concentrations ranging from 0.2 to 0.8% by weight.

19. A microemulsion as claimed in the above claims, wherein the viscosity-increasing agent is selected from a Carbomer such as Carbopol 941 and silicon dioxide.

20. A microemulsion as claimed in the above claims, wherein the absorption promoter is selected from glyceryl monooleate, limonene and decyl methyl sulphoxide.

21. Process for the preparation of the microemulsions as claimed in the above claims, comprising the following steps:
a) preparing an aqueous mixture containing one or more cosurfactants and one or more hydrophilic antioxidants, and optionally, one or more surfactants;
b) preparing a mixture containing at least one oil, at least one surfactant, one or more cosurfactants and one or more lipophilic antioxidants;
c) adding apomorphine, in the form of the hydrochloride or other salt, to the aqueous mixture, or in the form of the free base to the oily mixture;
d) contacting the oily mixture with the aqueous mixture under gentle stirring and at room temperature, to obtain a clear system;
e) optionally adding a viscosizing agent and an absorption promoter to the microemulsion obtained in step d).

22. The use of a microemulsion as claimed in the above claims, for the preparation of a medicament for use in all the treatments wherein the administration of apomorphine is required.

23. The use as claimed in claim 22 for the treatment of Parkinson's disease.

## Patentansprüche

1. Mikroemulsion, die Apomorphin oder Salze davon enthält und zur transdermalen Verabreichung geeignet ist.

2. Mikroemulsion nach Anspruch 1, bestehend aus
a) einem wässrigen Gemisch, das ein Co-Tensid oder mehrere Co-Tenside und ein hydrophiles Antioxidans oder mehrere hydrophile Antioxidanzien und gegebenenfalls ein Tensid oder mehrere Tenside enthält;
b) einem Gemisch, das mindestens ein Öl, mindestens ein Tensid, ein Co-Tensid oder mehrere Co-Tenside, ein lipophiles Antioxidans oder mehrere lipophile Antioxidanzien und andere Adjuvanzien enthält;
c) Apomorphin in Form des Hydrochlorids oder eines anderen Salzes;
d) gegebenenfalls die Viskosität erhöhende Mittel und Absorptionsverstärker.

3. Mikroemulsion nach den obigen Ansprüchen, wobei der pH im Bereich von 5,4 bis 6,2 liegt.

4. Mikroemulsion nach den obigen Ansprüchen, wobei der pH im Bereich von 5,6 bis 6,0 liegt.

5. Mikroemulsion nach den obigen Ansprüchen, wobei die Nanotropfen der dispergierten Phase einen Durchmesser im Bereich von 60 bis 100 nm haben.

6. Mikroemulsion nach den obigen Ansprüchen, wobei die Nanotropfen der dispergierten Phase einen Durchmesser im Bereich von 70 bis 90 nm haben.

7. Mikroemulsion gemäß den obigen Ansprüchen, wobei die Nanotropfen einen Polydispersitätsindex im Bereich von 0,10 bis 0,35 haben.

8. Mikroemulsion gemäß den obigen Ansprüchen, wobei das Öl aus aliphatischen Alkoholen, Estern aliphatischer Alkohole, Glycerinestern und Gemischen davon ausgewählt ist.

9. Mikroemulsion nach Anspruch 8, wobei das Öl aus aliphatischen Alkoholen mittlerer Kettenlänge und Estern davon, insbesondere Decanol, Dodecanol, Isopropylmyristat und Gemischen davon ausgewählt ist.

10. Mikroemulsion gemäß den obigen Ansprüchen, wobei das Tensid aus Polyoxyethylensorbitanen, Polyoxyethylencastorölderivaten, Sojalecithin, gereinigtem Eilecithin, Phospholipiden und Gemischen davon ausgewählt sind.

11. Mikroemulsion nach Anspruch 10, wobei die Tenside aus Tween 20, Tween 40, Tween 60, Cremophor EL ausgewählt sind.

12. Mikroemulsion gemäß den obigen Ansprüchen, wobei die Co-Tenside aus Alkoholen und Glykolen z.B. Butanol, Hexanol, Benzylalkohol, Cyclohexanol, Phenylethylalkohol, Proplyenglykol, Hexandiol, Gallensäuresalzen wie z.B. von Taurocholsäure und Glykocholsäure, Carbonsäuren wie Buttersäure, Hexansäure, Octansäure oder Salzen davon, Monoalkylphosphaten wie Octylphosphat und Hexadecylphosphat ausgewählt sind.

13. Mikroemulsion nach Anspruch 12, wobei die Co-Tenside aus Butanol, Hexanol, Benzylalkohol, Cyclohexanol, Phenylethylalkohol, Propylenglykol, Hexandiol ausgewählt sind.

14. Mikroemulsion nach Anspruch 12, wobei die Co-Tenside aus Taurocholsäure und Glykocholsäure ausgewählt sind.

15. Mikroemulsion nach Anspruch 12, wobei Co-Tenside aus Buttersäure, Hexansäure, Octansäure, oder Salzen derselben ausgewählt sind.

16. Mikroemulsion gemäß den obigen Ansprüchen, wobei die Antioxidanzien lipophiler und/oder hydrophiler Natur sind.

17. Mikroemulsion nach Anspruch 16, wobei die Antioxidanzien δ-Tocopherol, Ascorbylpalmitat, Ascorbinsäure sind.

18. Mikroemulsion gemäß den obigen Ansprüchen, wobei lipophile Antioxidanzien in Konzentrationen im Bereich von 0,2 bis 2,0 Gew.-% zugesetzt werden und hydrophile Antioxidanzien in Konzentrationen im Bereich von 0,2 bis 0,8 Gew.-% zugesetzt werden.

19. Mikroemulsion gemäß den obigen Ansprüchen, wobei das die Viskosität erhöhende Mittel aus einem Carbomer, z.B. Carbopol 941 und Siliziumdioxid ausgewählt ist.

20. Mikroemulsion gemäß den obigen Ansprüchen, wobei der Absorptionspromoter aus Glycerylmonooleat, Limonen und Decylmethylsulfoxid ausgewählt ist.

21. Verfahren zur Herstellung der Mikroemulsionen gemäß den obigen Ansprüchen, umfassend die folgenden Schritte:
a) Herstellen eines wässrigen Gemisches, das ein Co-Tensid oder mehrere Co-Tenside und ein hydrophiles Antioxidans oder mehrere hydrophile Antioxidanzien und gegebenenfalls ein Tensid oder mehrere Tenside enthält;
b) Herstellen eines Gemisches, das mindestens ein Öl, mindestens ein Tensid, ein Co-Tensid oder mehrere Co-Tenside, ein lipophiles Antioxidans oder mehrere lipophile Antioxidanzien enthält;
c) Zugeben von Apomorphin in Form des Hydrochlorids oder eines anderen Salzes zu dem wässrigen Gemisch oder in Form der freien Base zu dem öligen Gemisch;
d) In-Kontaktbringen des öligen Gemisches mit dem wässrigen Gemisch unter schonendem Rühren und bei Raumtemperatur, um ein klares System zu erhalten;
e) Gegebenenfalls Zusetzen eines die Viskosität erhöhenden Mittels und eines Absorptionspromotors zu der in Schritt d) erhaltenen Mikroemulsion.

22. Verwendung einer Mikroemulsion gemäß den obigen Ansprüchen zu der Herstellung eines Medikaments zur Verwendung in allen Behandlungen, bei denen die Verabreichung von Apomorphin erforderlich ist.

23. Verwendung nach Anspruch 22 zur Behandlung der Parkinsonerkrankung.

## Revendications

1. Microémulsion contenant de l'apomorphine ou des sels de celle-ci, appropriée pour l'administration transdermique.

2. Microémulsion selon la revendication 1, constituée par :
a) un mélange aqueux contenant un ou plusieurs co-agents tensioactifs et un ou plusieurs antioxydants hydrophiles et, facultativement, un ou plusieurs agents tensioactifs ;
b) un mélange contenant au moins une huile, au moins un agent tensioactif, un ou plusieurs co-agents tensioactifs, un ou plusieurs antioxydants lipophiles et d'autres adjuvants ;
c) de l'apomorphine, sous la forme du chlorhydrate ou d'un autre sel ;
d) facultativement, des agents augmentant la viscosité et des activateurs d'absorption.

3. Microémulsion selon les revendications ci-dessus, dans laquelle le pH va de 5,4 à 6,2.

4. Microémulsion selon les revendications ci-dessus, dans laquelle le pH est dans la gamme de 5,6 à 6,0.

5. Microémulsion selon les revendications ci-dessus, dans laquelle les nanogouttes de la phase dispersée ont un diamètre allant de 60 à 100 nm.

6. Microémulsion selon les revendications ci-dessus, dans laquelle les nanogouttes de la phase dispersée ont un diamètre allant de 70 à 90 nm.

7. Microémulsion selon les revendications ci-dessus, dans laquelle les nanogouttes ont un indice de polydispersité allant de 0,10 à 0,35.

8. Microémulsion selon les revendications ci-dessus, dans laquelle l'huile est choisie parmi les alcools aliphatiques, les esters d'alcools aliphatiques, les esters du glycérol et des mélanges de ceux-ci.

9. Microémulsion selon la revendication 8, dans laquelle l'huile est choisie parmi les alcools aliphatiques à chaîne moyenne et les esters de ceux-ci, en particulier le décanol, le dodécanol, le myristate d'isopropyle et des mélanges de ceux-ci.

10. Microémulsion selon les revendications ci-dessus, dans laquelle les agents tensioactifs sont choisis parmi les polyoxyéthylène sorbitanes, les dérivés polyoxyéthylénés d'huile de ricin, la lécithine de soja, la lécithine d'oeuf purifiée, les phospholipides et des mélanges de ceux-ci.

11. Microémulsion selon la revendication 10, dans laquelle les agents tensioactifs sont choisis parmi le Tween 20, le Tween 40, le Tween 60, le Cremophor EL.

12. Microémulsion selon les revendications ci-dessus, dans laquelle les co-agents tensioactifs sont choisis parmi les alcools et les glycols, comme le butanol, l'hexanol, l'alcool benzylique, le cyclohexanol, l'alcool phényléthylique, le propylèneglycol, l'hexanediol, les sels biliaires tels que taurocholique et glycocolique, les acides carboxyliques tels que les acides butyrique, hexanoïque, octanoïque ou des sels de ceux-ci, les phosphates de monoalkyle tels que le phosphate d'octyle et le phosphate d'hexadécyle.

13. Microémulsion selon la revendication 12, dans laquelle les co-agents tensioactifs sont choisis parmi le butanol, l'hexanol, l'alcool benzylique, le cyclohexanol, l'alcool phényléthylique, le propylèneglycol, l'hexanediol.

14. Microémulsion selon la revendication 12, dans laquelle les co-agents tensioactifs sont choisis parmi taurocholique et glycocolique.

15. Microémulsion selon la revendication 12, dans laquelle les co-agents tensioactifs sont choisis parmi l'acide butyrique, hexanoïque, octanoïque ou des sels de ceux-ci.

16. Microémulsion selon les revendications ci-dessus, dans laquelle les agents antioxydants sont de nature lipophile et/ou hydrophile.

17. Microémulsion selon la revendication 16, dans laquelle les agents antioxydants sont le δ-tocophérol, le palmitate d'ascorbyle, l'acide ascorbique.

18. Microémulsion selon les revendications ci-dessus, dans laquelle les agents antioxydants lipophiles sont ajoutés en des concentrations allant de 0,2 à 2,0% en poids, et des antioxydants hydrophiles sont ajoutés en des concentrations allant de 0,2 à 0,8 % en poids.

19. Microémulsion selon les revendications ci-dessus, dans laquelle l'agent augmentant la viscosité est choisi parmi un Carbomer tel que le Carbopol 941 et le dioxyde de silicium.

20. Microémulsion selon les revendications ci-dessus, dans laquelle le promoteur d'absorption est choisi parmi le monooléate de glycéryle, le limonène et le sulfoxyde de décyle et de méthyle.

21. Procédé pour la préparation des microémulsions selon les revendications ci-dessus, comprenant les étapes suivantes :
a) préparation d'un mélange aqueux contenant un ou plusieurs co-agents tensioactifs et un ou plusieurs antioxydants hydrophiles et, facultativement, un ou plusieurs agents tensioactifs ;
b) préparation d'un mélange contenant au moins une huile, au moins un agent tensioactif, un ou plusieurs co-agents tensioactifs et un ou plusieurs antioxydants lipophiles ;
c) ajout d'apomorphine, sous la forme du chlorhydrate ou d'un autre sel, au mélange aqueux, ou sous la forme de la base libre au mélange huileux ;
d) mise en contact du mélange huileux avec le mélange aqueux sous agitation douce et à température ambiante, pour obtenir un système transparent ;
e) facultativement, ajout d'un agent de viscosité et d'un activateur d'absorption à la microémulsion obtenue dans l'étape d).

22. Utilisation d'une microémulsion selon les revendications précédentes, pour la préparation d'un médicament pour l'utilisation dans tous les traitements dans lesquels l'administration d'apomorphine est requise.

23. Utilisation selon la revendication 22 pour le traitement de la maladie de Parkinson.
